# EUROPEAN PATENT SPECIFICATION

(11) **EP 2 194 772 B1**
(45) Date of publication and mention of the grant of the patent: **10.09.2014**
(21) Application number: 08806303.7
(22) Date of filing: 17.09.2008
(51) Int. Cl.: A01H 4/00, A01H 5/00, A01H 5/10, C12N 15/82

(54) **TRANSFORMATION OF POINSETTIA PLANTS**
TRANSFORMATION VON POINSETTIA-PFLANZEN
TRANSFORMATION DE POINSETTIAS

(30) Priority: 17.09.2007 GB 0718112
(43) Date of publication of application: 16.06.2010
(73) Proprietor: Norwegian Institute For Agricultural&Environmental Research, 1432 Aas (NO)
(72) Inventor: CLARKE, Jihong, Liu, N-1511 Moss (NO); BLYSTAD, Dag, Ragnar, N-1430 Aas (NO); HAUGSLIEN, Sissel, Britt, N-1820 Spydeberg (NO)
(74) Representative: Webber, Philip Michael
(86) International application number: PCT/GB2008/003147
(87) International publication number: WO 2009/037443

(56) References cited:
- WO-A-99/06566
- US-A1- 2007 083 948
- CLOSE K R ET AL: "STRUCTURE-ACTIVITY RELATIONSHIPS OF AUXIN-LIKE PLANT GROWTH REGULATORS AND GENETIC INFLUENCES ON THE CULTURE INDUCTION RESPONSE IN MAIZE ZEA-MAYS L" PLANT SCIENCE (SHANNON), vol. 61, no. 2, 1989, pages 245-252, XP023485230 ISSN: 0168-9452
- JIHONG LIU CLARKE ET AL: "Agrobacterium tumefaciens-mediated transformation of poinsettia, Euphorbia pulcherrima, with virus-derived hairpin RNA constructs confers resistance to Poinsettia mosaic virus" PLANT CELL REPORTS, SPRINGER, BERLIN, DE, vol. 27, no. 6, 8 March 2008 (2008-03-08), pages 1027-1038, XP019626103 ISSN: 1432-203X

## Description

The invention relates to a process for the Agrobacterium-mediated transformation of *Euphorbia pulcherrima* (Poinsettia). Also disclosed are *Euphorbia pulcherrima* plants which have been transformed in this way and to plant material obtained therefrom.

Poinsettia *(Euphorbia pulcherrima* Willd. Ex Klotzsch) is a contemporary symbol of Christmas in most parts of the world. Consumption of flowers in Norway continues to increase 5-10 % per year. Ornamentals produced in greenhouses have a value of approximately 40 % of the total first-hand value of the production in horticulture in Norway (Statistics 2003-2004 from the Norwegian Grower's Association (NGF)). In Norway, poinsettia is one of the most important pot plants with a yearly production close to 6 million plants. The annual production in the USA and the EU is 50 million and 100 million plants, respectively. Today, Europe and North America represent the largest volume of production and sales, but demand is growing quickly in Australasia as poinsettia becomes more popular each year (Williams 2005). Its ornamental value and innovation potential has laid the basis for extensive research in Norway.

For many years, the focus from both the grower's association (G3 Ungplanter) and academia has been on creating new cultivars with improved ornamental values such as enhanced quality and resistance to diseases in existing poinsettia. Traditionally, the improvement of quality and ornamental values for poinsettia has been achieved by conventional breeding methods. Breeders have managed to provide new varieties in flower colour, vigour, leaf form, and pest and disease resistance etc. using known methods. However, traditional breeding is limited to variations which exist in the gene pools of the parent species; these are not enough to meet the markets' demands. Sexual incompatibility is another factor restricting the utilization of genetic resources by traditional breeding approaches. With advances in technology, a number of new techniques, such *as in vitro* breeding techniques, embryo rescue, *in vitro* pollination, protoplast fusion and mutagenesis have been developed to complement the weaknesses of conventional breeding. However, another common disadvantage of both traditional and *in vitro* breeding techniques is the long, time-consuming backcrossing process. This can often take many years and has limited the number of commercially-viable cultivars to date.

Genetic transformation technology can overcome the limitations of the gene pool, sexual barriers and the necessity for subsequent backcrossing. Genetic transformation has been utilized in crop improvement conferring important agronomic traits including disease resistance against various pathogens, in addition to many other important traits. This technology overcomes the difficulties encountered by traditional approaches and can directly introduce desirable genes/agronomic traits into a target plant efficiently.

Viral diseases in plants can cause serious economic losses every year. Unlike fungal diseases where chemical method can be used to successfully prevent diseases, control of plant virus diseases has been more problematic. Control strategies have consisted mainly of approaches aimed at reducing or eliminating existing sources of infection within or outside the target crop, prevention of virus transmission, or minimizing the effects of infection. Cross protection and breeding for resistant or tolerant cultivars have been the main approaches applied worldwide. For ornamental plants, genetic transformation has been utilized in many flowers with desirable horticultural properties and enhanced ornamental values.

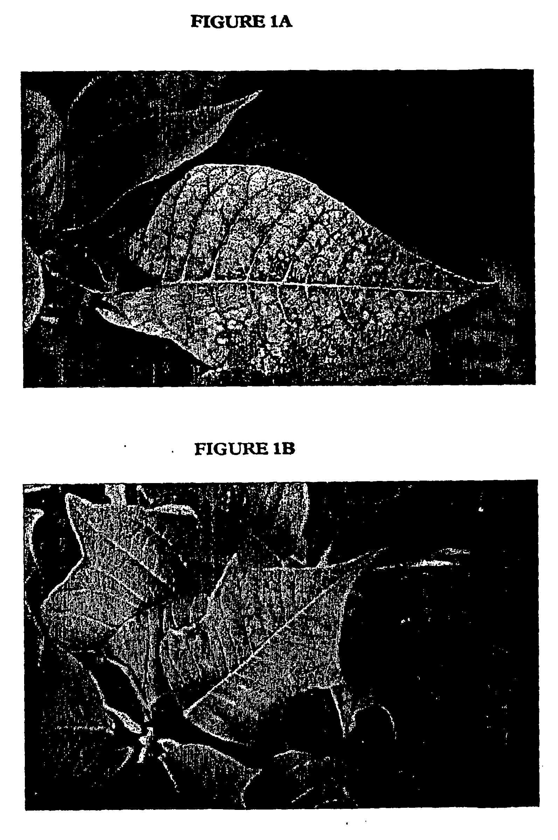

Poinsettia Mosaic Virus (PnMV) is a disease which causes damage to poinsettia cultivars. PnMV causes visible symptoms in poinsettia during parts of the growing season. These symptoms consist of light green areas - mosaics - in the leaves (Fig. 1). This mosaicing is most pronounced in the green leaves on the border to the coloured top leaves (Fig. 1).

Therefore there is considerable interest in the potential benefits of growing PnMV-free poinsettias. Traditionally, PnMV-free poinsettia plants were obtained by *in vitro* culture of apical meristems. However, it is a time-consuming and labour intensive method.

Conditions for the transformation of the cells of many plants with *Agrobacterium* are well known in the art. A protocol for Poinsettia transformation has been described in US 2007/0083948. In light of this, biolistic approaches for the transformation of Poinsettia have been developed (e.g. US 7,119,262). However, this has several disadvantages, e.g. random integration of the transgene, multiple copies of the transgene, gene silencing, instability of transgene etc.

To overcome the difficulties encountered by traditional approaches and some of the disadvantages of the biolistic method, an *Agrobacterium*-mediated transformation method for *Euphorbia pulcherrima* has at last been developed. The methodology developed facilitates the improvement of ornamental *Euphorbia pulcherrima* plants with aims to enhance their disease resistance, quality traits and ornamental values.

The invention therefore relates to a process for producing a transformed *Euphorbia pulcherrima* plant, comprising transforming *Euphorbia pulcherrima* plant material with *Agrobacterium* containing a gene construct and regenerating a transformed *Euphorbia pulcherrima* plant from the transformed plant material.

In one embodiment, the invention provides a process for producing a transgenic *Euphorbia pulcherrima* somatic embryo, comprising the steps of:
(a) culturing a transformable *Euphorbia pulcherrima* plant material, wherein the plant material is a plant cell, plant tissue, explant or protoplast, on or in a callus inducing media comprising CPA and BAP;
(b) inoculating the transformable *Euphorbia pulcherrima* plant material with *Agrobacterium* which contains a gene construct comprising a selectable marker gene and a reporter gene and which is capable of transferring the gene construct to the plant material;
(c) culturing the transformable *Euphorbia pulcherrima* plant material after inoculation with *Agrobacterium* on or in a callus inducing media comprising CPA and BAP in the absence of the selection agent and an anti-*Agrobacterium* agent;
(d) culturing the transformable *Euphorbia pulcherrima* plant material on or in a callus inducing media comprising CPA and BAP in the presence of an *anti-Agrobacterium* agent and the selection agent;
(e) culturing the embyrogenic calli thus produced on or in a somatic embryo induction media comprising NAA and 2iP in the presence of an anti-*Agrobacterium* agent and the selection agent;
(f) culturing the somatic embryos thus produced on or in a somatic embryo induction media comprising NAA and 2iP in the presence of an anti-*Agrobacterium* agent and the selection agent, and optionally subculturing the somatic embryos one or more additional times.

In some embodiments, the process additionally includes the step:
(g) culturing the somatic embryos on or in a somatic embryo maturation media comprising BAP.

In other embodiments, the process includes the additional steps:
(g) culturing the somatic embryos on or in a somatic embryo maturation media comprising BAP; and
(h) culturing plantlets derived from the somatic embryos on or in root induction media.

Preferably, the *Euphorbia is Euphorbia pulcherrima* Willd. Ex Klotzsch (poinsettia).

A particularly preferred *Euphorbia pulcherrima* is *Euphorbia pulcherrima* (cv. Millennium). Other examples of *Euphorbia pulcherrima cultivars* include Cristella, Angelika, Lilo and Cortez.

In the context of the present invention, the term "transformable *Euphorbia pulcherrima* plant material" refers to a *Euphorbia pulcherrima* plant cell, plant tissue, explant or plant protoplast which is capable of being transformed with a genetic construct, such as a Ti-DNA or derivative thereof, from an *Agrobacterium* species.

Examples of plant cells, plant tissues, explants or plant protoplasts include *inter alia* leaves, leaf explants, stems, stem explants, shoot apices, roots, hypocotyls, cotyledons, seeds, pollen, calli, immature and somatic embryos, shoot apical meristems, etc.

Preferably, the plant material is a plant cell, callus, immature or somatic embryo, shoot apical meristem, leaf explant, stem explant or protoplast.

Examples of cells include cells obtained from the aforementioned materials and calli derived from various parts of the plant. Examples of explants includes explants obtained from the aforementioned materials.

The plant from which the plant material is obtained is preferably a young plant, more preferably one which is 4-12 weeks old, most preferably one which is 6-8 weeks old.

In some embodiments, the plant material is a stem, leaf, zygotic embryo, somatic embryo, embryogenic calli, apical meristem, or a cell obtained therefrom or suspension of cultured cells or protoplasts.

Preferably, the plant tissue which is transformed is a stem explant, most preferably an internode stem explant, and particularly preferably a stem explant which has been taken from right below the shoot apical meristem. Preferably, the stem explants are 0.5-1.5 cm long.

Prior to use, the plant material may be sterilized in order to limit undesirable bacterial growth. For example, stem explants may be sterilized using alcohol and/or NaOCl, followed by rinsing with water.

In Step (a), prior to transformation with *Agrobacterium,* the plant material is cultured on or in callus inducing (CI) media comprising CPA, and BAP.

The general composition of "callus inducing (CI) media" will be well known to persons skilled in the art. One example of such media is MS basal medium comprising macro and micro components and minerals which are necessary for plant growth. (For medium suitable for poinsettia, reference is: Preil, W. 1994. In vitro culture of poinsettia,. In E. Strømme (ed.). The scientific basis of poinsettia production. Agricultural University of Norway.)

However, general CI media have previously not enabled the preparation of transformable *Euphorbia pulcherrima* plant material. The inventors have found that callus inducing (CI) media comprising CPA and BAP is efficacious in this regard.

Preferably, the CI media comprises 0.1-0.35 mg L⁻¹ CPA, more preferably 0.15-0.25 mg L⁻¹ CPA, and most preferably about 0.2 mg L⁻¹ CPA.

Preferably, the CI media comprises 0.1-0.35 mg L⁻¹ BAP, more preferably 0.15-0.25 mg L⁻¹ BAP, and most preferably about 0.2 mg L⁻¹ BAP.

The time that the plant material may be cultured on or in callus inducing (CI) media comprising CPA and BAP is preferably 20-28 hours, more preferably 22-26 hours, and most preferably about 1 day.

The plant material is preferably cultured on or in callus inducing (CI) media comprising CPA and BAP in the dark or under reduced light conditions.

The *Agrobacterium* used in the invention is one which contains a gene construct comprising a selectable marker gene and a reporter gene and which is capable of transferring the gene construct to the plant material.

Preferably the *Agrobacterium* is *Agrobacterium tumefaciens.*

The skilled person will understand that, in order to facilitate the transfer of the gene construct to the plant material, the vir gene must also be present in the *Agrobacterium,* either in the gene construct or in a separate plasmid/vector. Preferably, the *Agrobacterium* is an *Agrobacterium tumefaciens* which contains a disarmed Ti plasmid which has only the *vir* and *ori* region of the Ti plasmid.

Most preferably, the *Agrobacterium* is *Agrobacterium tumefaciens* strain LBA 4404.

The "gene construct comprising a selectable marker gene and a reporter gene" will generally be a nucleic acid vector or nucleic acid plasmid. The skilled person will be familiar with vectors and plasmids which are based upon or derived from the Ti (tumour inducing) plasmid, a large conjugative plasmid naturally found in *Agrobacterium tumefaciens.*

Within the Ti plasmid is a segment of DNA known as the T-DNA which is flanked by T-DNA borders. Upon transformation of the Ti plasmid into the plant, the T-DNA is inserted into the genome of the plant. This provides a route for inserting a foreign or heterologous nucleic acid molecules into the genome of the plant.

The gene construct comprises at least a selectable marker gene and a reporter gene. In other embodiments, the reporter gene is a selectable marker gene.

In one embodiment, the gene construct is a plasmid or vector which comprises a selectable marker gene and a reporter gene flanked by at least one T-DNA border. Preferably, the {selectable marker gene and reporter gene} are flanked by two T-DNA borders. Where only one T-DNA border is present, the right-hand border is present.

In certain embodiments, the gene construct comprises:
(i) a left *Agrobacterium* T-DNA border sequence;
(ii) a selectable marker gene comprising:
   a promoter which functions in *Euphorbia pulcherrima* plant cells
   a nucleotide sequence encoding a selectable marker polypeptide
   a 3' non-translated region encoding a terminator sequence
(iii) a reporter gene comprising:
   a promoter which functions in *Euphorbia pulcherrima* plant cells
   a nucleotide sequence of interest
   a 3' non-translated region encoding a terminator sequence
(iv) a right *Agrobacterium* T-DNA border sequence.

In other embodiments, the order of the selectable marker gene and the reporter gene are reversed.

The promoter may be any plant promoter which is operable in *Euphorbia pulcherrima.* The preferred promoter is CaMV 35S promoter.

The selectable marker gene encodes a polypeptide which confers resistance to the selection agent. The selection agent is, for example, an antibiotic suitable for plant cell culture; it might also be a herbicide.

Preferably, the selectable marker gene is neomycin phosphotransferase II *(nptII)* and the selection agent is kanamycin. Examples of other suitable selectable marker genes/selection agents include hygromycin and the herbicide resistance gene *bar.* Other selection marker genes include *Escherichia coli* aspartate kinase or *LysC,* the green fluorescence protein (gfp) and the luciferase gene (reviewed by Stewart 2001, The utility of green fluorescent protein in transgenic plants. Plant Cell Rep. 20: 376-382, incorporated herein by reference).

The nucleic acid molecule of interest in the reporter gene may comprise one or more of the following, *inter alia:*
- A disease resistance gene or pathogenesis related (PR) protein-encoding gene, for example a gene which encodes polypeptide with an anti-bacterial, anti-fungal, insecticidal or anti-viral properties.
- A gene which encodes a polypeptide which modifies a known phenotypic trait or introduces a new phenotypic trait to the plant. Examples of such traits include increased leaf production and increased branching, and tolerance to coldness or dryness.
- A gene which encodes a polypeptide which modifies the colour of all or part of the plant or affects the ornamental value of the plant. The skilled person will be aware of the considerable demand for *Euphorbia,* e.g. Poinsettia, which are yellow, pure white, blue or lilac. For colour or quality improvement, the appropriate gene which is responsible for the target trait is used. For example, for producing yellow-coloured poinsettia, the colour biosynthetic pathway may be altered by expressing two transgenes (AmAS1 and Am4'CGT).
- A gene-silencing construct, for example one comprising a region of first DNA and a region of second DNA which is complementary to the first DNA, the first and second DNAs being separated by an intron or spacer. Upon transcription, the gene-silencing construct makes a hairpin loop structure. Gene-silencing constructs may particularly be used to stop and/or reduce infection by generating small interfering RNAs (siRNAs) which are homologous to parts of the genome of one or more viruses.

Further examples of nucleic acid molecules of interest include those encoding antibodies, antibiotics, herbicides, vaccine antigens, enzymes, enzyme inhibitors and designer peptides.

A preferred nucleic acid molecule of interest is one which encodes a fragment of the Poinsettia Mosaic Virus (PnMV) coat protein. Preferably, the nucleic acid molecule of interest encodes a fragment of the Poinsettia Mosaic Virus (PnMV) coat protein in sense and antisense orientations, separated by an intron or spacer. Preferably the intron is a *pdk* intron. Preferably, the fragment is about 500 nucleotides in length.

A further preferred nucleic acid molecule of interest is one which encodes a fragment of an RNA dependent RNA polymerase. Preferably, the nucleic acid molecule of interest encodes a fragment of an RNA dependent RNA polymerase in sense and antisense orientations, separated by an intron or spacer. Preferably the intron is a *pdk* intron. Preferably, the fragment is about 500 nucleotides in length.

The gene construct may also contain other elements which enable its handling and reproduction, such as an origin of replication, selection elements, and multiple cloning sites.

Preferably, the gene construct is a pHANNIBAL or pKANNIBAL vector (CSIRO).

*Agrobacterium* containing the gene construct may be prepared by standard means. In general, the steps will involve the preparation of competent *Agrobacterium* cells; and the introduction of the gene construct into *Agrobacterium,* for example by electroporation.

*Agrobacteria* containing the gene construct may be grown overnight in an appropriate liquid media, e.g. LB media (Sigma), supplemented with an appropriate selection agent.

The *Agrobacteria* are preferably grown in the liquid media with shaking at 150-250 rpm, more preferably at 175-225 rpm, and most preferably at about 200 rpm.

The *Agrobacteria* are preferably grown overnight at a temperature of 25-31 °C, more preferably at a temperature of 27-29 °C, and most preferably at a temperature of about 28 °C.

The *Agrobacieria* are grown to an appropriate OD, preferably to OD₆₀₀=0.5-1.0, more preferably 0.55-0.65, most preferably about 0.6.

The selection agent, preferably an antibiotic, is preferably used at a concentration of 25-75 mg L⁻¹, more preferably at a concentration of 40-60 mg L⁻¹, and most preferably at a concentration of about 50 mg L⁻¹.

Once grown, the *Agrobacterium* may be separated from the liquid media, for example, by pelleting using centrifugation.

The *Agrobacterium* are then washed to remove traces of the overnight growth media and then resuspended in a suitable media prior to inoculation of the plant material. Preferably, the *Agrobacterium* are washed with Murashige & Skoog (MS) basal medium supplemented with about 2% sucrose (MS-2), and resuspended in MS-2.

In Step (b), the transformable *Euphorbia pulcherrima* plant material is inoculated with *Agrobacterium* which contains a gene construct comprising a selectable marker gene and a reporter gene. The *Agrobacterium* is one which is capable of transferring the gene construct to the plant material.

As used herein, the term "inoculating" means infecting a *Euphorbia pulcherrima* plant material or incubating protoplasts from the *Euphorbia pulcherrima* plant with *Agrobacterium.*

This inoculation is carried out under conditions which are suitable for the gene construct contained within the *Agrobacterium* to be transferred to the plant material.

The *Agrobacterium* (preferably resuspended in MS-2) are applied to the plant material for a time which allows transfer of the gene construct to occur.

In one embodiment, the *Agrobacterium* are co-cultivated with the plant material for a time which allows the *Agrobacterium to* attach to the plant material. Preferably this time is 2-20 minutes, more preferably 5-15 minutes and most preferably about 5 minutes.

In Step (c), the transformable *Euphorbia pulcherrima* plant material are cultured after inoculation on or in a callus inducing (CI) media comprising CPA and BAP in the absence of the selection agent. Furthermore, the CI media should not contain an *anti-Agrobacterium* agent. At this time, the *Agrobacterium* have attached themselves to the plant material and the physical transfer of the gene construct occurs.

Previous general CI media have not enabled the *Agrobacterium-mediated* transformation of *Euphorbia pulcherrima.* The inventors have found that callus inducing (CI) media comprising CPA and BAP is efficacious in this regard.

The term "anti-*Agrobacterium* agent" refers to an agent which kills *Agrobacterium* or reduces significantly the activity of the *Agrobacterium.*

The *Agrobacterium* are preferably co-cultured with the plant material at a temperature of 20-28 °C, more preferably 22-26 °C and most preferably at about 24 °C. Preferably, they are co-cultured for 24-120 hours, more preferably for 48-96 hours, and most preferably for about 72 hours. It is preferred that this co-culturing is carried out in the dark or under reduced light conditions.

In Step (d), after transformation with *Agrobacterium,* the plant tissue is cultured on or in CI media comprising CPA and BAP in the presence of an anti-*Agrobacterium* agent and the selection agent.

Preferably, the anti-*Agrobacterium* agent is claforan. Preferably, the concentration of the anti-*Agrobacterium* agent is 300-500 mg L⁻¹, and most preferably a concentration of about 400mg L⁻¹.

The time that the plant material may be cultured on or in the above-defined callus inducing (CI) media is preferably 2-15 days, more preferably 5-12 hours, and most preferably 7-10 days. After this time, embryogenic calli are produced.

Preferably, the light conditions are 20-26 µE m⁻² s⁻¹, most preferably about 23 µE m⁻² s⁻¹. The photoperiod is preferably 14-18 hours, and most preferably about 16 hours.

In Step (e), the embryogenic calli are cultured on or in a somatic embryo inducing media comprising NAA and 2iP in the presence of an anti-*Agrobacterium* agent and the selection agent.

The general composition of the "somatic embryo inducing media" which is usable in this regard will be well known to persons skilled in the art. One example of such a media is MS basal medium comprising a given percent sucrose content and appropriate hormones.

However, general somatic embryo inducing media have previously not enabled the induction of somatic embryos from transformed *Euphorbia pulcherrima* plant material. The inventors have found that somatic embryo inducing media comprising NAA and 2iP in the presence of an anti-*Agrobacterium* agent and the selection agent is efficacious in this regard.

Preferably, the somatic embryo inducing media comprises 0.1-0.4 mg L⁻¹ NAA, more preferably 0.25-0.35 mg L⁻¹ NAA, and most preferably about 0.3 mg L⁻¹ NAA.

Preferably, the somatic embryo inducting media comprises 0.10-0.30 mg L⁻¹ 2iP, more preferably 0.12-0.20 mg L⁻¹ 2iP, and most preferably about 0.15 mg L⁻¹ 2iP.

Preferably the *anti-Agrobacterium* agent is claforan. Preferably the concentration of the *anti-Agrobacterium* agent is 300-500 mg L⁻¹, and most preferably at a concentration of about 400mg L⁻¹.

The time that the embryogenic calli may be cultured on or in the above-defined somatic embryo induction media is preferably 10-18 days, more preferably 12-16 days, and most preferably about 14 days. After this time, somatic embryogenic calli are produced.

Preferably, the light conditions are 20-26 µE m⁻² s⁻¹, most preferably about 23 µE m⁻² s⁻¹. The photoperiod is preferably 14-18 hours, most preferably about 16 hours.

In Step (f), the embryogenic calli are sub-cultured further on or in a somatic embryo inducing media comprising NAA, 2iP, an anti-*Agrobacterium* agent and the selection agent.

If the selection marker gene is *nptII,* the selection agent may be kanamycin. Preferably, the concentration of selection agent used in this Step is 10-40 mg L-¹, more preferably 20-30 mg L⁻¹ and most preferably about 25 mg L⁻¹.

The *anti-Agrobacterium* agent is preferably claforan. Preferably, the concentration of anti-*Agrobacterium* agent used in this Step is 300-700 mg L⁻¹, more preferably 400-600 mg L⁻¹, and most preferably about 500 mg L⁻¹.

The somatic embryos may then be subcultured, for example a further 2-4 times. The globular stage of the somatic embryos becomes visible on the calli after 2-3 subcultures, i.e. after 4-6 weeks. In the further subcultures, the concentration of selection marker may be reduced. For example, if the selection marker gene is *nptII,* the selection agent may be kanamycin. Preferably, the concentration of kanamycin is reduced to 3-20 mg L⁻¹, more preferably 5-15 mg L⁻¹, and most preferably about 10 mg L⁻¹.

Preferably, the concentration of anti-*Agrobacterium* agent (e.g. claforan) is reduced to 200-300 mg L⁻¹, more preferably to 225-275 mg L⁻¹, and most preferably to about 250 mg L⁻¹.

Somatic embryos develop after an average of about 10 weeks. These may be transferred to somatic embryo maturation (SEM) media comprising BAP.

The general composition of some "somatic embryo maturation media" will be well known to persons skilled in the art. One example of such media is MS basal medium and a given percent sucrose content. The inventors have found that somatic embryo maturation (SEM) media comprising BAP is particularly efficacious in this regard.

Preferably, the somatic embryo maturation media comprises 0.03-0.07 mg L⁻¹ BAP, more preferably 0.04-0.06 mg L⁻¹ BAP, and most preferably about 0.05 mg L⁻¹ BAP.

Preferably, the light conditions are 20-26 µE m⁻² s⁻¹, most preferably about 23 µE m⁻2 s⁻¹.

Plantlet derived from somatic embryos may subsequently be cultured on root induction media. Such media are well known in the art.

Examples of root induction media are ½ strength MS comprising 2 mg L⁻¹ IAA and hormone-free ½ strength MS (HFMS) medium.

Preferably, the light conditions are 20-40 µE m⁻² s⁻¹, most preferably about 30 µE m⁻² s⁻¹. The photoperiod is preferably 14-18 hours, most preferably about 16 hours.

Plants with well-developed roots may then be transferred to soil and optionally grown in greenhouses at about 22°C.

The invention also provides a process for producing a transgenic Euphorbia *pulcherrima* plant, comprising the steps of:
(a) culturing a transformable *Euphorbia pulcherrima* plant material, wherein the plant material is a plant cell, plant tissue, explant or protoplast, on or in a callus inducing media comprising CPA and BAP;
(b) inoculating the transformable *Euphorbia pulcherrima* plant material with *Agrobacterium* which contains a gene construct comprising a selectable marker gene and a reporter gene and which is capable of transferring the gene construct to the plant material;
(c) culturipg the transformable *Euphorbia pulcherrima* plant material after inoculation with *Agrobacterium* on or in a callus inducing media comprising CPA and BAP in the absence of the selection agent and an anti-*Agrobacterium* agent;
(d) culturing the transformable *Euphorbia pulcherrima* plant material on or in a callus inducing media comprising CPA and BAP in the presence of an anti-Agrobacterium agent and the selection agent;
(e) culturing the embyrogenic calli thus produced on or in a somatic embryo induction media comprising NAA and 2iP in the presence of an anti-*Agrobacterium* agent and the selection agent;
(f) culturing the somatic embryos thus produced on or in a somatic embryo induction media comprising NAA and 2iP in the presence of an anti-Agrobacterium agent and the selection agent, and optionally subculturing the somatic embryos one or more additional times;
(g) culturing the somatic embryos on or in a somatic embryo maturation media comprising BAP; and
(h) culturing plantlets derived from the somatic embryos on or in root induction media in order to produce a transgenic *Euphorbia* plant.

The disclosure also provides a *Euphorbia pulcherrima* somatic embryo or *Euphorbia pulcherrima* plant obtained by or obtainable by a process of the invention. Also disclosed is plant material (for example, cuttings, flowers, bushes and trees) obtained from or obtainable from the aforementioned *Euphorbia pulcherrima* somatic embryos or *Euphorbia pulcherrima* plants.

In particular, the disclosure encompasses transgenic seeds and cuttings, and meristems by *in vitro* culture obtained from or obtainable from the aforementioned *Euphorbia pulcherrima* somatic embryos or *Euphorbia pulcherrima* plants which comprise the gene construct.

The disclosure also provides a *Euphorbia pulcherrima* plant, plant cell, plant tissue or plant protoplast which comprises in its genome a region of DNA having the nucleotide sequence of at least one T-DNA border.

Preferably, the T-DNA border is a right T-DNA border.

The *Euphorbia pulcherrima* plant, plant cell, plant tissue or plant protoplast comprises in its genome a genetic construct comprising a selectable marker and reporter gene flanked by one or more T-DNA borders.

The disclosure also relates to plant material (for example, cuttings, flowers, bushes and trees) obtained from or obtainable from the aforementioned *Euphorbia pulcherrima* plants, plant cells or plant tissues

In particular, the disclosure encompasses transgenic seeds and cuttings, meristems by in vitro culture obtained from or obtainable from the aforementioned *Euphorbia pulcherrima* plants, plant cells or plant tissue which comprise the genetic construct.

### Brief description of Figures

Figures 1A and 1B. Poinsettia cv. Millennium showing mosaic symptoms caused by poinsettia mosaic virus (PnMV).
Figure 2: Diagrammatic representation of the three hpRNA constructs.
   CP: coat protein region (500 bp in length).
   R2 and R3: RNA dependent RNA polymerase (RdRp) regions about 500 bp in length.
Figures 3A-C: Somatic embryogenesis derived from Agrobacterium-mediated transformation in poinsettia cv. Millennium.

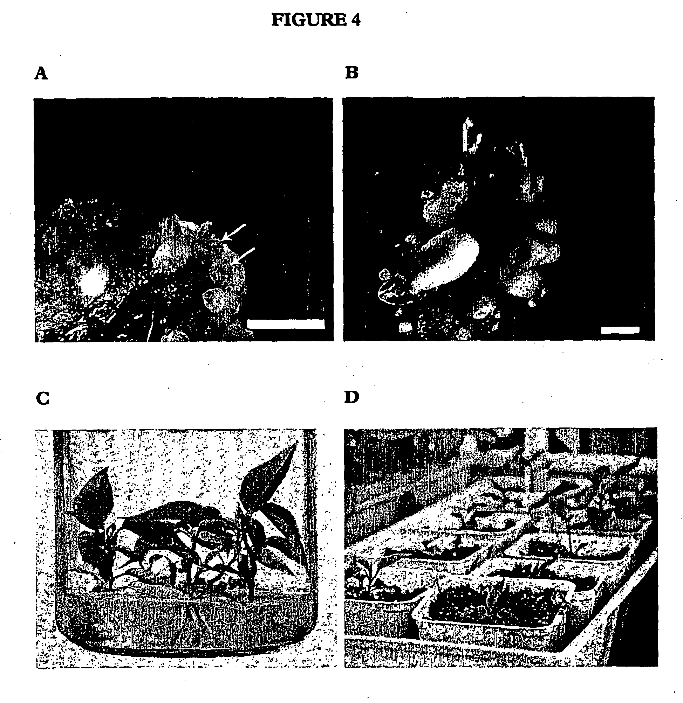

Fig. 4: Somatic embryogenesis derived from Agrobacterium-mediated transformation in poinsettia cv. Millennium.
   (A) Embryogenic structure and globular stage somatic embryos (arrows) appeared on the callus after four weeks of culture (bar: 1mm).
   (B) Cotyledonary stage of somatic embryos (bar: 1 mm).
   (C) Plantlets deriving from somatic embryos on RIM medium.
   (D) Regenerated plants established in the greenhouse.

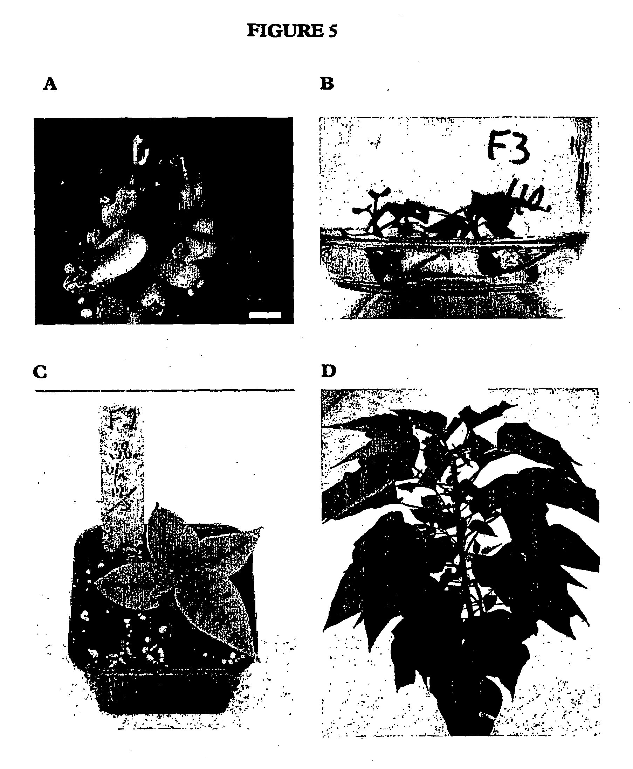

Fig. 5: Agrobacterium-mediated transformation of poinsettia cv. Millennium.
   (A) Somatic embryos induced from internode explants after transformation.
   (B) Regenerated poinsettia after kanamycin selection.
   (C) and (D) Transgenic poinsettia plants in the greenhouse.

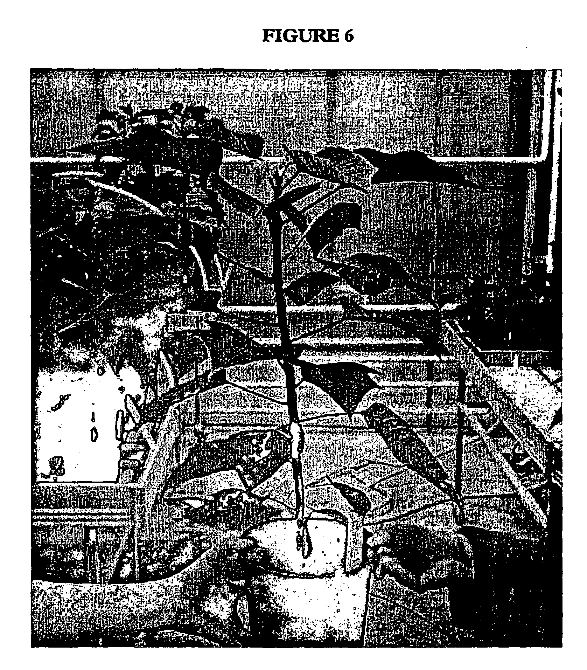

Fig. 6: A poinsettia transformant growing in the greenhouse.

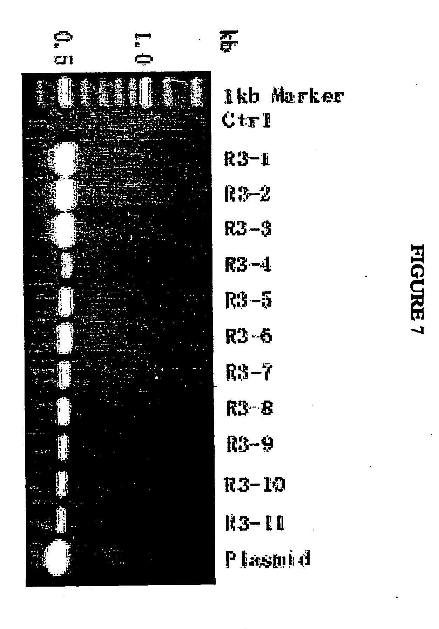

Fig. 7: PCR screening of DNA samples from transgenic plants with the vector pR3 construct. Ctrl: untransformed plants serving as control. Plasmid: positive control. Lanes form R3-1 to R3-11 are the putative transgenic plants.

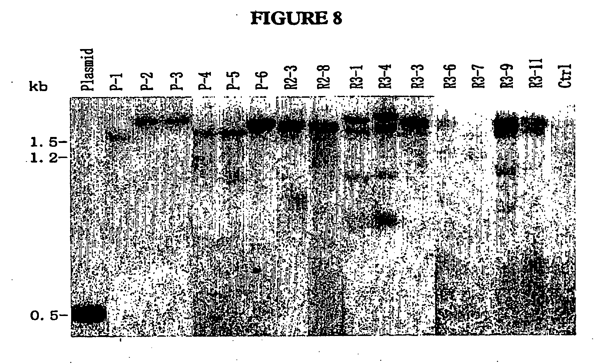

Fig. 8: Southern analysis of *Hind*III digested genomic DNA from transgenic poinsettia and control. Ctrl: untransformed control; Plasmid: plasmid DNA as positive control; Lane 2-15: poinsettia transformants.

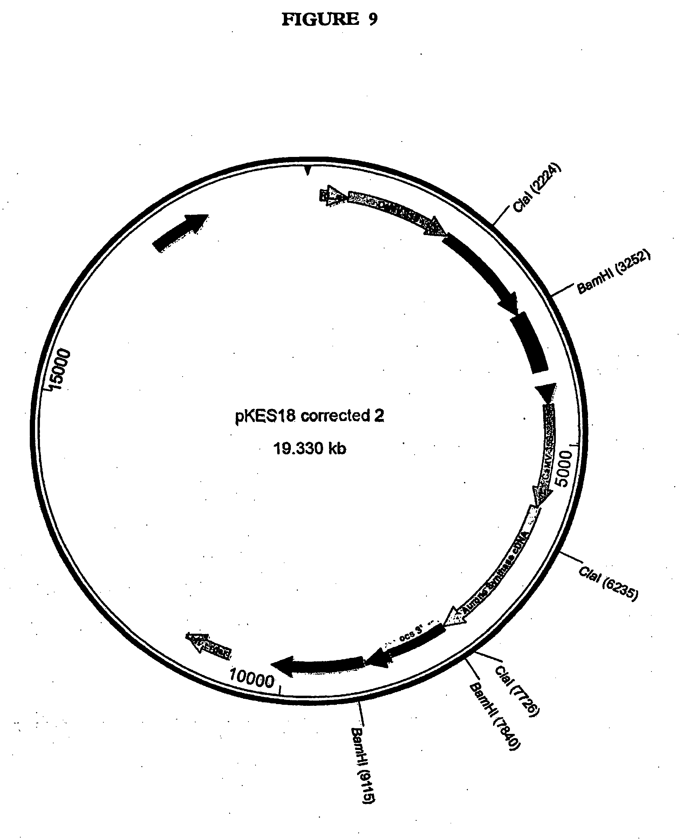

Fig. 9: Plasmid pKES18 corrected 2.

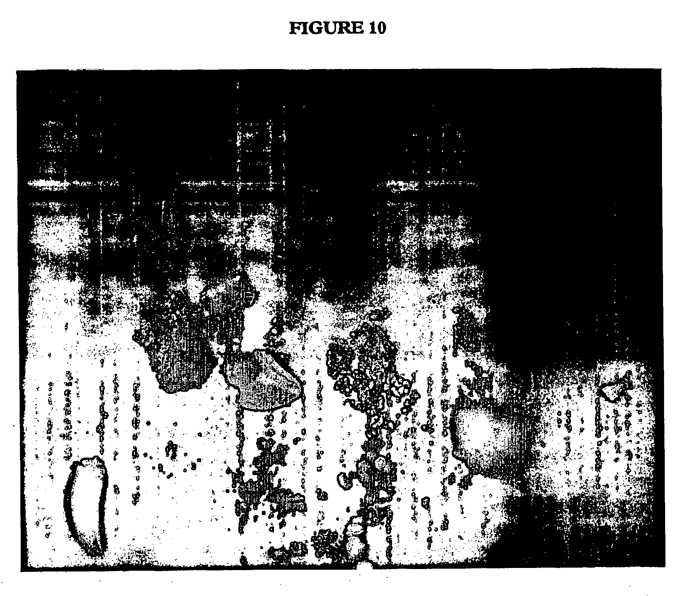

Fig. 10: PCR-positive transgenic Poinsettia cv. Cristella embryos.

### EXAMPLES

The following abbreviations are used herein:
- 2iP: (2-isopentenyl)adenine
- BAP: 6-benzylaminopurine
- CI: callus induction medium
- CP: coat protein
- CPA: (4-chlorophenoxy)acetic acid
- HSFM: hormone free half strength MS medium
- IAA: indole-3 acetic acid
- MS: Murashige and Skoog medium (1962)
- MS-2: MS medium + 2% sucrose
- NAA: 1-naphthaleneacetic acid
- PnMV: Poinsettia mosiac virus
- RdRp: RNA dependent RNA polymerase
- RI: root induction medium
- SEI: somatic embryo induction medium
- SEM: somatic embryo maturation medium

### Derivatives

Whilst specific reference is made herein to the above media and products, the invention also encompasses the use of derivatives of the above media/products and equivalents thereof. For example, whilst reference is made herein to 2iP, BAP, CAP, IAA and NAA, the invention also relates to derivatives (for example acid addition salts) and obvious variants which have the same or equivalent biological effect.

The present invention is further defined in the following.Examples, in which parts and percentages are by weight and degrees are Celsius, unless otherwise stated. It should be understood that these Examples, while indicating preferred embodiments of the invention, are given by way of illustration only.

**Table 1:**

| **Media names and compositions** | | | | | | | |
|---|---|---|---|---|---|---|---|
| Medium | MS | Sucrose | CPA | BAP | NAA | 2iP | IAA |
| CI | Full strength | 3% | 0.2 mg/l | 0.2 mg/l | - | - | - |
| SEI | Full strength | 3% | - | - | 0.3 mg/l | 0.15 mg/l | - |
| SEM | Full strength | 3% | - | 0,05 mg/l | - | - | - |
| RI | ½ strength | 2% | - | - | - | - | 2 mg/l |
| HFMS | ½ strength | 2% | - | - | - | - | - |

### Example 1:

### Preparation of plant material

*Euphorbia pulcherrima* cv. Millennium plants were kindly supplied by Kristiansen nursery, Grimstad, Norway, in the year 2000. The original stock plants were subjected to heat therapy to eliminate poinsettia mosaic virus (PnMV). PnMV-free Millennium cuttings were grown in the greenhouse under a photoperiod of 16 h light and 8 h dark with a temperature of 22°C. Internode stem explants from eight to ten weeks old Millennium plants were used for *Agrobacterium*-mediated transformation of poinsettia.

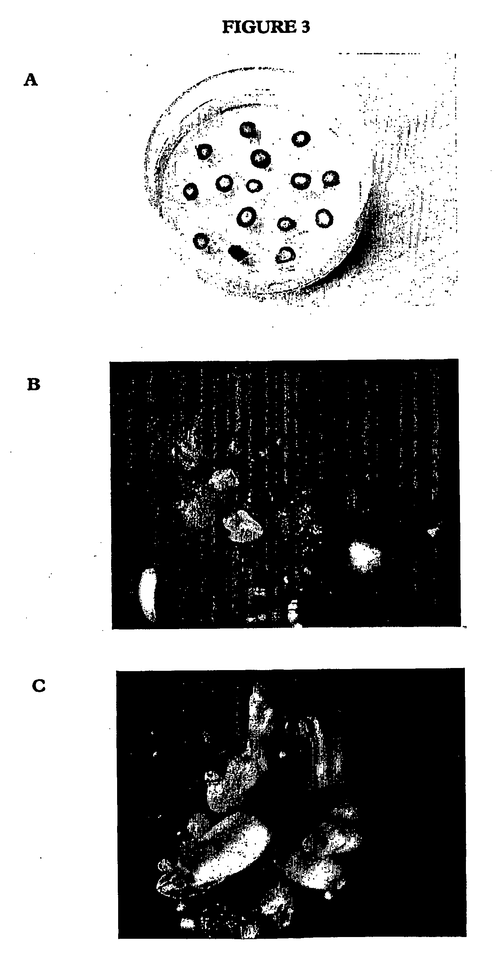

Stem explants 0.5-1.5 cm long taken below the shoot apical meristem from Millennium plants were excised and used in the present study (Fig. 3). The stem explants were sterilized with an alternative method: 70% alcohol for 1 min followed by 5 min with 1% NaOCl and 3 rinsings with sterile deionized and autoclaved water for 3, 10 and 20 min. After sterilization, stem explants were placed on callus induction (CI) medium containing 0.2 mg L⁻¹ CPA and 0.2 mg L⁻¹ BAP (Table 1) at 24 °C in dark for one day before *Agrobacterium* inoculation.

### Example 2:

### Agrobacterium strains and hairpin (hp) RNA constructs

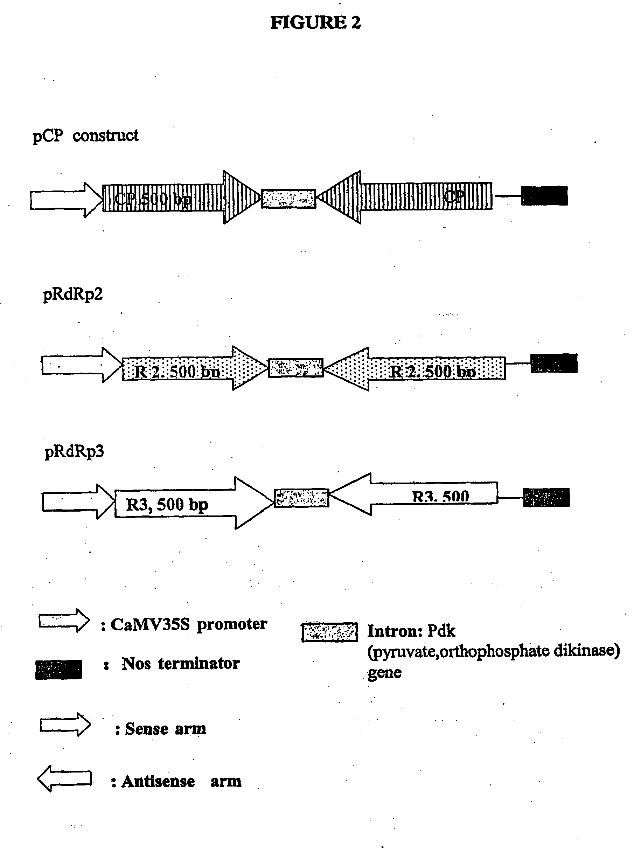

*Agrobacterium tumefaciens* strain LBA4404 (Hoekema et al. 1983, Nature 303:179-181); Invitrogen), harbouring three hairpin (hp) RNA constructs, namely pCP, pR2 and pR3, derivatives of the original pHANNIBAL. provided by CSIRO Plant Industry (Canberra, Australia), was used in all the experiments. The constructs were cloned into pART27, a binary plasmid at *Not1* site. The diagrammatic presentations of those hpRNA constructs are shown in Fig. 2. The pCP construct contains a 500 bp fragment of coat protein (CP) gene inserted in sense and antisense orientations interrupted by a intron *pdk* (pyruvate,orthophosphate dikinase) gene, whereas the pR2 and pR3 constructs, possessing gene fragments (entitled R2 and R3) of RNA dependent RNA polymerase (RdRp) region about 500 bp in length and interrupted by the same *pdk* gene respectively (Fig. 2). CP, R2 and R3 fragments were amplified by PCR, cloned into *E. coli* before being ligated into the defined restriction sites flanking the intron *pdk* gene. All the three expression cassettes were under the control of the CaMV 35S promoter. For selection, the neomycin phosphotransferase II (*nptII*) gene conferring kanamycin resistant was used under the control of nopaline synthase promoter (Nos-P).

### Example 3:

### Agrobacterium-mediated transformation

*A. tumefaciens* strain LBA4404, carrying the plasmid pCP, pR2 and pR3, was grown overnight in 15 ml liquid LB medium (Sigma, USA) supplemented with 50 mg L⁻¹ kanamycin (Duchefa) at 28 °C with shaking at 200 rpm until an OD600=0.6 was reached. The bacterium suspension was pelleted at 2700 rpm for 10 min, washed with Murashige & Skoog (MS, Murashige & Skoog, 1962, A revised medium for rapid growth and bioassays with tobacco tissue cultures. Physiol. Plant 15(3):473-497) basal medium supplemented with 2% sucrose (MS-2), and resuspended in 10 ml MS-2.

The pre-prepared stem explants excised into stem segments with 1-1.5 mm thickness (Fig. 2) were infected with *Agrobacterium* suspension for 5 min with gentle shaking by hand, blotted briefly with sterile filter paper (Walkman), and placed on CI medium (Table 1) at 24°C in dark for 72h without selection. Subsequently, the explants were blotted gently on sterile filter paper and transferred to the CI medium supplemented with 400 mg L⁻¹ claforan.

### Example 4:

### Somatic embryogenesis and regeneration of transgenic plants

After 7-10 days on CI medium supplemented with 400 mg L⁻¹ claforan, embryogenic calli appeared on brownish stem segment explants were transferred to somatic embryo induction (SEI) medium containing 0.3 mg L⁻¹ NAA and 0.15 mg L⁻¹ 2iP (Table 1) supplemented with 400 mg L⁻¹ claforan for somatic embryogenesis for 2 weeks. After the first round sub-culture, embryogenic calli were transferred to SEI medium supplemented with 500 mg L⁻¹ claforan and 25 mg L⁻¹ kanamycin. The globular stage of the somatic embryos became visible on the calli after 2-3 round sub-cultures, i.e. after 4-6 weeks. After further a couple of subcultures, the SEI medium was supplemented with 250 mg L⁻¹ claforan and 10 mg L⁻¹ kanamycin. The somatic embryos developed after 10 weeks were then transferred to somatic embryo maturation (SEM) medium containing 0.05 mg L⁻¹ BAP (Table 1). Plantlets derived from somatic embryos were subsequently cultured on root induction (RI) medium containing ½ strength MS and 2 mg L⁻¹ IAA or hormone free ½ strength MS (HFMS) medium (Table 1) for root induction. Plants with well-developed roots were transferred to soil and grown in the greenhouse at 22 °C.

Light conditions were 23µE m⁻² s⁻¹ for the callus and somatic embryos on CI, SEI and SEM media, whereas 30µE m⁻² s⁻¹ for plantlets in RIM and HFMS media with a 16h photoperiod. Light microscopy connected with a digital camera was used to follow up the development of somatic embryos.

### Example 5:

### Polymerase chain reaction (PCR) for screening or transgenic poinsettia lines

Three primer pairs designed for amplifying the CP, R2 and R3 fragments were:
CP: forward
   5' acgtctagaAACCACGTCGACTCCACTCCAT 3' (SEQ ID NO:1)
CP: reverse
   5' agcatcgatAGCTTGCCGCTCACCAGCAC 3' (SEQ ID NO:2)
R2: forward
   5' acgtctagaTTTAGCAAAACGCAGCACAAAATCA 3' (SEQ ID NO:3)
R2: reverse
   5'agcatcgatTCTCCAGACACCATGATTGGGTG 3' (SEQ ID NO:4)
R3: forward
   5' acgtctagaTTCGCTTTAAAACAGAAAGCACCA 3' (SEQ ID NO:5)
R3: reverse
   5' agcatcgatGCCTCGTAGCTTGGTTGGGTT 3' (SEQ ID NO:6)

The HotStarTaq PCR kit purchased from Qiagen (Valencia, California) and standard PCR kit from Applied Biosystems (manufactured by Roche Molecular Systems, Inc., New Jersey, USA) were utilized in PCR screening of transformants. For HotStartTaq PCR kit, 20 µl reaction mixture containing 2x HotstarTaq Mastermix, 0.4 µM of each primer, 0.1 µg template DNA and H2O was subjected to PCR amplification with following conditions: 15 min at 95 °C (1 cycle), 30 sec at 95 °C, 30 sec at 55 °C, 1 min at 72 °C (35 cycles) and a final extension 10 min at 72 °C (1 cycle). When the standard PCR kit was used, a 20 µl reaction mixture consisted of 10x PCR buffer, 3 mM MgCl, 250 µM each of dNTPs, 0.4 µM of each primer, and 0.1 µg template DNA and 1 U *Taq* DNA polymerase. The amplification was performed in Applied Biosystems 96 Thermal Cycler (Applied Biosystems) with program: 5 min at 95 °C (1 cycle), 30 sec at 95 °C, 30 sec at 56 °C, and 1 min at 72 °C (35 cycles), and a final elongation 7 min at 72 °C (1 cycle). The PCR fragments produced were separated on a 0.8% (W/V) agarose gel and evaluated.

### Example 6:

### Southern blot analysis of transformants

For Southern blot analysis, total genomic DNA was isolated from young leaves of control and transformed plants following a modified CTAB protocol as described by Rogers SO, Bendich AJ (1988) (Extraction of DNA from plant tissues. In: Gelvin SB, Schilperoort RA (eds) Plant Molecular Biology Manual, pp A6:1-10. Boston, MA: Kluwer. Academic Publishers). Ten micrograms of genomic DNA was digested with the restriction enzymes *Hind*III and *Eco*RI respectively for 4 h and separated on a 1% (W/V) TBE agarose gel overnight at 37 V. After transfer to a nylon membrane, the blot was prehybridized and hybridized with ³²P labelled probe of 1.5 kb in size consisted of the three 500 bp fragments, i.e. CP, R2 and R3. Southern blot hybridization was performed and membranes were washed and exposed to X-ray films basically as described by Sambrook et al. (1989) (Molecular cloning: A laboratory manual, 2nd ed., Cold Spring Harbor Laboratory Press, Plainview, NY).

### Example 7:

### Resistance assay after inoculation with poinsettia mosaic virus (PnMV)

Transgenic poinsettia lines were multiplied vegetatively using cuttings. The rooted cuttings were inoculated by sap inoculation at 3-4 leaves stage. The inoculum consisted of a mixture of PnMV multiplied in *Nicotiana benthamiana* and in *Poinsettia pulcherrima* diluted about 1:5 w/v. The inoculated plants were tested for PnMV several times by ELISA during the 4-6 months observation period.

### Example 8:

### Elisa assays

Enzyme-linked immunosobent assay, ELISA, was performed with antisera from DSMZ (Deutsche Sammlung von Mikroorganismen und Zellkulturen GmbH, Braunschewig, Germany), according to the manufacturer's instructions.

After inoculation with virus, leaf samples from both inoculated and new leaves were harvested several times during the following growth and observation period.

Based on PCR and Southern blot analyses, transgenic poinsettia lines by *Agrobacterium-*mediated transformation were obtained (Fig. 5a and 5b). Stable integrations of transgenes were confirmed by Southern blot analysis (Fig. 7). Inoculation experiment with PnMV followed by Elisa assays revealed PnMV resistance in transgenic poinsettia lines.

### Example 9:

### Transformation of Poinsettia cv. "Cristella"

The Agrobacterium-mediated transformation of *Euphorbia pulchenima* cv. Cristella was performed based on our protocol (Clarke et al. 2008, Plant Cell Rep. 27:1027-1038, the contents of which are incorporated in entirety). *A. tumefaciens* strain LBA4404 was used, harbouring the plasmid pKES18 (Figure 9) which contains the *A. majus* aureusidin synthase (AmAS1) and chalcone 4'-O-glucosyltransferase (4'CGT) genes for production of yellow-colored poinsettia (Ono et al. 2006, PNAS vol. 103, no. 29: 11075-11080, the contents of which are incorporated in entirety).

Internode stem explants of Cristella taken from 8-10 week old poinsettia plants derived from cuttings were disinfected as described in Example 1, excised into stem segments with 1-1.5 mm thickness and inoculated with *Agrobacterium* suspension for 5 min with gentle shaking. All the procedures including infection, co-cultivation, selection and regeneration were identical to that for Millennium as described above. PCR-positive transgenic Cristella embryos are shown in Figure 10.

### SEQUENCE LISTING FREE TEXT

SEQ ID NO: 1
   <223> Forward PCR primer used to amplify CP fragment
SEQ ID NO: 2
   <223> Reverse PCR primer used to amplify CP fragment
SEQ ID NO: 3
   <223> Forward PCR primer used to amplify R2 fragment
SEQ ID NO: 4
   <223> Reverse PCR primer used to amplify R2 fragment
SEQ ID NO: 5
   <223> Forward PCR primer used to amplify R3 fragment
SEQ ID NO: 6
   <223> Reverse PCR primer used to amplify R3 fragment

## Claims

1. A process for producing a transgenic *Euphorbia pulcherrima* somatic embryo, comprising the steps of:
(a) culturing a transformable *Euphorbia pulcherrima* plant material, wherein the plant material is a plant cell, plant tissue, explant or protoplast, on or in a callus inducing media comprising CPA and BAP;
(b) inoculating the transformable *Euphorbia pulcherrima* plant material with *Agrobacterium* which contains a gene construct comprising a selectable marker gene and a reporter gene and which is capable of transferring the gene construct to the plant material;
(c) culturing the transformable *Euphorbia pulcherrima* plant material after inoculation with *Agrobacterium* on or in a callus inducing media comprising CPA and BAP in the absence of the selection agent and an anti*-Agrobacterium* agent;
(d) culturing the transformable *Euphorbia pulcherrima* plant material on or in a callus inducing media comprising CPA and BAP in the presence of an anti-*Agrobacterium* agent and the selection agent;
(e) culturing the embyrogenic calli thus produced on or in a somatic embryo induction media comprising NAA and 2iP in the presence of an anti-*Agrobacterium* agent and the selection agent;
(f) culturing the somatic embryos thus produced on or in a somatic embryo induction media comprising NAA and 2iP in the presence of an anti-*Agrobacterium* agent and the selection agent, and optionally subculturing the somatic embryos one or more additional times.

2. A process as claimed in claim 1, which additionally comprises the step:
(g) culturing the somatic embryos on or in a somatic embryo maturation media comprising BAP.

3. A process as claimed in claim 1 or claim 2, which additionally comprises the step:
(h) culturing plantlets derived from the somatic embryos on or in root induction media.

4. A process as claimed in any one of the preceding claims, wherein the *Euphorbia pulcherrima* is *Euphorbia pulcherrima* Willd. Ex Klotsch (poinsettia), preferably wherein the *Euphorbia pulcherrima* cultivar is Millennium, Cristella, Angelika, Lilo or Cortez.

5. A process as claimed in any one of the preceding claims, wherein the plant material is a plant cell, callus, immature or somatic embryo, shoot apical meristem, leaf explant, stem explant or protoplast, preferably wherein the plant material is a stem explant.

6. A process as claimed in any one of the preceding claims, wherein the plant from which the plant material has been obtained is 6-8 weeks old.

7. A process as claimed in any one of the preceding claims, wherein in Step (a):
(i) the CI media comprises 0.1-0.35 mg L⁻¹ CPA; or
(ii) the CI media comprises 0.1-0.35 mg L⁻¹ BAP; or
(iii) the plant material is cultured on or in callus inducing (CI) media comprising CPA and BAP for 20-28 hours; or
(iv) the plant material is cultured on or in callus inducing (CI) media comprising CPA and BAP in the dark or under reduced light conditions.

8. A process as claimed in any one of the preceding claims, wherein the *Agrobacterium* is *Agrobacterium tumefaciens,* preferably wherein the *Agrobacterium* is *Agrobacterium tumefaciens* strain LBA 4404.

9. A process as claimed in any one of the preceding claims, wherein the gene construct is a DNA plasmid or vector which comprises a selectable marker gene and a reporter gene flanked by at least one T-DNA border.

10. A process as claimed in any one of the preceding claims, wherein the gene construct comprises:
(i) a left *Agrobacterium* T-DNA border sequence;
(ii) a selectable marker gene comprising:
a promoter which functions in *Euphorbia pulcherrima* plant cells
a nucleotide sequence encoding a selectable marker polypeptide
a 3' non-translated region encoding a terminator sequence
(iii) a reporter gene comprising:
a promoter which functions in *Euphorbia pulcherrima* plant cells
a nucleotide sequence of interest
a 3' non-translated region encoding a terminator sequence
(iv) a right *Agrobacterium* T-DNA border sequence.

11. A process as claimed in any one of the preceding claims, wherein the selectable marker gene encodes a polypeptide which confers resistance to the selection agent.

12. A process as claimed in any one of the preceding claims, wherein the selection agent is an antibiotic or a herbicide, preferably wherein the selectable marker gene is neomycin phosphotransferase II (nptII) and the selection agent is kanamycin.

13. A process as claimed in any one of the preceding claims, wherein the reporter gene comprises:
- a disease resistance gene or pathogenesis related (PR) protein-encoding gene;
- a gene which encodes a polypeptide which modifies a known phenotypic trait or introduces a new phenotypic trait to the plant;
- a gene which encodes a polypeptide which modifies the colour of all or part of the plant or affects the ornamental value of the plant; or
- a gene-silencing construct,
preferably wherein the reporter gene comprises a nucleic acid molecule which encodes a fragment of the Poinsettia Mosaic Virus (PnMV) coat protein or wherein the reporter gene comprises a nucleic acid molecule which encodes a fragment of an RNA dependent RNA polymerase.

14. A process as claimed in any one of the preceding claims, wherein:
(i) prior to Step (b), the *Agrobacteria* are grown to an appropriate OD₆₀₀=0.5-1.0; or
(ii) in Step (b), the *Agrobacterium* are co-cultivated with the plant material for 2-20 minutes, more preferably 5-15 minutes and most preferably about 5 minutes.

15. A process as claimed in any one of the preceding claims, wherein in Step (c):
(i) the *Agrobacterium* are co-cultured with the plant material at a temperature of 20-28°C, more preferably 22-26°C; or
(ii) the *Agrobacterium* are co-cultured with the plant material at for 24-120 hours, more preferably for 48-96 hours; or
(iii) the *Agrobacterium* are co-cultured with the plant material in the dark or under reduced light conditions.

16. A process as claimed in any one of the preceding claims, wherein in Step (d):
(i) the concentration of the *anti-Agrobacterium* agent is 300-500 mg L⁻¹; or
(ii) the *anti-Agrobacterium* agent is claforan; or
(iii) the time that the plant material is cultured on or in the callus inducing (CI) media is 2-15 days, preferably 5-12 hours, and most preferably 7-10 days; or
(iv) the light conditions are 20-26 µE m⁻² s⁻¹; or
(v) the photoperiod is 14-18 hours.

17. A process as claimed in any one of the preceding claims, wherein in Step (e):
(i) the somatic embryo inducing media comprises 0.1-0.4 mg L⁻¹ NAA, preferably 0.25-0.35 mg L⁻¹ NAA; or
(ii) the somatic embryo inducing media comprises 0.10-0.30 mg L⁻¹ 2iP, preferably 0.12-0.20 mg L⁻¹ 2iP; or
(iii) the concentration of the anti*-Agrobacterium* agent is 300-500 mg L⁻¹; or
(iv) the anti*-Agrobacterium* agent is claforan; or
(v) the time that the embryogenic calli is cultured on or in the somatic embryo induction media is 10-18 days, preferably 12-16 days; or
(vi) the light conditions are 20-26 µE m⁻² s⁻¹; or
(vii) the photoperiod is 14-18 hours; or
(viii) the concentration of selection agent used is 10-40 mg L⁻¹, more preferably 20-30 mg L⁻¹.

18. A process as claimed in any one of the preceding claims, wherein in Step (f):
(i) the concentration of anti*-Agrobacterium* agent used is 300-700 mg L⁻¹, preferably 400-600 mg L⁻¹; or
(ii) the *anti-Agrobacterium* agent is claforan; or
(iii) the somatic embryos are optionally subcultured a further 2-4 times.

## Patentansprüche

1. Verfahren zur Herstellung eines transgenen somatischen Embryos von *Euphorbia pulcherrima,* das die Schritte umfasst:
(a) Kultivieren von transformierbarem Pflanzenmaterial von *Euphorbia pulcherrima,* wobei das Pflanzenmaterial eine Pflanzenzelle, Pflanzengewebe, ein Explantat oder Protoplast ist, auf oder in einem Kallus induzierenden Medium, das CPA und BAP enthält;
(b) Inokulieren des transformierbaren Pflanzenmaterials von *Euphorbia pulcherrima* mit *Agrobacterium,* das ein Genkonstrukt enthält, das ein selektierbares Markergen und ein Reportergen umfasst und dazu in der Lage ist, das Genkonstrukt in das Pflanzenmaterial zu transferieren;
(c) Kultivieren des transformierbaren Pflanzenmaterials von *Euphorbia pulcherrima* nach der Inokulation mit *Agrobacterium* auf oder in einem Kallus induzierenden Medium, das CPA und BAP in Anwesenheit des Selektionswirkstoffs und einen Wirkstoff gegen *Agrobacterium* enthält;
(d) Kultivieren des transformierbaren Pflanzenmaterials von *Euphorbia pulcherrima* auf oder in einem Kallus induzierenden Medium, das CPA und BAP in Anwesenheit eines Wirkstoffs gegen *Agrobacterium* und des Selektionswirkstoffs enthält;
(e) Kultivieren der hierdurch produzierten embryogenen Kalli auf oder in einem somatische Embryonen induzierenden Medium, das NAA und 2iP in Anwesenheit eines Wirkstoffs gegen *Agrobacterium* und des Selektionswirkstoffs enthält;
(f) Kultivieren der hierdurch produzierten somatischen Embryonen auf oder in einem somatische Embryonen induzierenden Medium, das NAA und 2iP in Anwesenheit eines Wirkstoffs gegen *Agrobacterium* und des Selektionswirkstoffs enthält, und optional ein-, oder mehrmaliges zusätzliches Subkultivieren der somatischen Embryonen.

2. Verfahren nach Anspruch 1, das zusätzlich den Schritt umfasst:
(g) Kultivieren der somatischen Embryonen auf oder in einem Medium zur Ausreifung somatischer Embryonen, das BAP enthält.

3. Verfahren nach Anspruch 1 oder 2, das zusätzlich den Schritt umfasst:
(h) Kultivieren von Pflänzchen, die aus den somatischen Embryonen erhalten wurden, auf oder in einem Wurzeln induzierenden Medium.

4. Verfahren nach einem der vorstehenden Ansprüche, wobei die *Euphorbia pulcherrima Euphorbia pulcherrima* Willd. Ex Klotsch (Poinsettia) ist, wobei der Kultivar von *Euphorbia pulcherrima* vorzugsweise Millennium, Cristella, Angelika, Lilo oder Cortez ist.

5. Verfahren nach einem der vorstehenden Ansprüche, wobei das Pflanzenmaterial eine Pflanzenzelle, Kallus, ein unreifer oder somatischer Embryo, ein Sprossapikalmeristem, ein Blattexplantat, ein Stängelexplantat oder ein Protoplast ist, wobei das Pflanzenmaterial vorzugsweise ein Stängelexplantat ist.

6. Verfahren nach einem der vorstehenden Ansprüche, wobei die Pflanze, von der das Pflanzenmaterial erhalten wurde, 6 bis 8 Wochen alt ist.

7. Verfahren nach einem der vorstehenden Ansprüche, wobei in Schritt (a):
(i) das CI-Medium 0,1-0,35 mg L⁻¹ CPA enthält; oder
(ii) das CI-Medium 0,1-0,35 mg L⁻¹ BAP enthält; oder
(iii) das Pflanzenmaterial auf oder in einem Kallus induzierenden (CI) Medium, das CPA und BAP enthält, für 20 bis 28 Stunden kultiviert wird; oder
(iv) das Pflanzenmaterial auf oder in einem Kallus induzierenden (CI) Medium, das CPA und BAP enthält, im Dunklen oder unter reduzierten Lichtbedingungen kultiviert wird.

8. Verfahren nach einem der vorstehenden Ansprüche, wobei das *Agrobacterium Agrobacterium tumefaciens* ist, wobei das *Agrobacterium* vorzugsweise der *Agrobacterium tumefaciens-Stamm* LBA 4404 ist.

9. Verfahren nach einem der vorstehenden Ansprüche, wobei das Genkonstrukt ein DNA-Plasmid oder Vektor ist, der ein auswählbares Markergen und ein Reportergen aufweist, die durch wenigstens eine T-DNA-Grenze voneinander getrennt sind.

10. Verfahren nach einem der vorstehenden Ansprüche, wobei das Genkonstrukt aufweist:
(i) eine linke *Agrobacterium* T-DNA-Grenzsequenz;
(ii) ein auswählbares Markergen, das enthält:
einen in Pflanzenzellen von *Euphorbia pulcherrima* funktionsfähigen Promotor
eine Nukleotidsequenz, die für ein auswählbares Marker-Polypeptid kodiert
eine nichttranslatierte 3'-Region, die für eine Terminatorsequenz kodiert
(iii) ein Reportergen, das enthält:
einen in Pflanzenzellen von *Euphorbia pulcherrima* funktionsfähigen Promotor
eine Nukleotidsequenz von Interesse eine nichttranslatierte 3'-Region, die für eine Terminatorsequenz kodiert
(iv) eine rechte *Agrobacterium*-T-DNA-Grenzsequenz.

11. Verfahren nach einem der vorstehenden Ansprüche, wobei das selektierbare Markergen für ein Polypeptid kodiert, das eine Resistenz gegenüber dem Selektionswirkstoff bewirkt.

12. Verfahren nach einem der vorstehenden Ansprüche, wobei der Selektionswirkstoff ein Antibiotikum oder Herbizid ist, wobei das selektierbare Markergen Neomycin-Phosphotransferase II (nptII) ist und der Selektionswirkstoff Kanamycin ist.

13. Verfahren nach einem der vorstehenden Ansprüche, wobei das Reportergen umfasst:
- ein Krankheitsresistenzgen oder ein für ein mit Pathogenese in Beziehung stehendes (PR) Protein kodierendes Gen;
- ein Gen, das für ein Polypeptid kodiert, das ein bekanntes phänotypisches Merkmal modifiziert oder ein neues phänotypisches Merkmal in die Pflanze einbringt;
- ein Gen, das für ein Polypeptid kodiert, das die Farbe der gesamten oder eines Teils der Pflanze verändert oder einen Zierwert der Pflanze beeinflusst; oder
- ein ein Gen stilllegendes Konstrukt,
wobei das Reportergen vorzugsweise ein Nukleinsäuremolekül enthält, das für ein Fragment des Hüllproteins des Poinsettia-Mosaikvirus (PnMV) kodiert, oder wobei das Reportergen ein Nukleinsäuremolekül enthält, das für ein Fragment einer RNA-abhängigen Polymerase kodiert.

14. Verfahren nach einem der vorstehenden Ansprüche, wobei:
(i) vor Schritt (b) die Agrobakterien bis zu einer geeigneten OD₆₀₀ = 0,5 bis 1,0; oder
(ii) in Schritt (b) die Agrobakterien mit dem Pflanzenmaterial für 2 bis 20 Minuten, eher vorzugsweise 5 bis 15 Minuten und besonders vorzugsweise um 5 Minuten cokultiviert werden.

15. Verfahren nach einem der vorstehenden Ansprüche, wobei in Schritt (c):
(i) die Agrobakterien mit dem Pflanzenmaterial bei einer Temperatur von 20 bis 28 °C, eher vorzugsweise bei 22 bis 26 °C cokultiviert werden; oder
(ii) die Agrobakterien mit dem Pflanzenmaterial für 24 bis 120 Stunden, eher vorzugsweise für 48 bis 96 Stunden cokultiviert werden, oder
(iii) die Agrobakterien mit dem Pflanzenmaterial im Dunklen oder unter reduzierten Lichtbedingungen cokultiviert werden.

16. Verfahren nach einem der vorstehenden Ansprüche, wobei in Schritt (d):
(i) die Konzentration des Wirkstoffs gegen Agrobacterium 300 bis 500 mg L⁻¹ beträgt,
(ii) der Wirkstoff gegen *Agrobacterium* Claforan ist; oder
(iii) die Zeit, für die das Pflanzenmaterial auf oder in dem Kallus induzierenden (CI) Medium kultiviert wird, 2 bis15 Tage, vorzugsweise 5 bis 12 Stunden und besonders vorzugsweise 7 bis 10 Tage beträgt; oder
(iv) die Lichtbedingungen 20 bis 26 µE m⁻² s⁻¹ sind, oder
(v) die Photoperiode 14 bis 18 Stunden betrage.

17. Verfahren nach einem der vorstehenden Ansprüche, wobei in Schritt (e):
(i) das somatische Embryonen induzierende Medium 0,1 bis 0,4 mg L⁻¹ NAA, vorzugsweise 0,25 bis 0,35 mg L¹ NAA enthält; oder
(ii) das somatische Embryonen induzierende Medium 0,10 bis 0,30 mg L⁻¹ 2iP, vorzugsweise 0,12 bis 0,20 mg L⁻¹ 2iP enthält; oder
(iii) die Konzentration des Wirkstoffs gegen *Agrobacterium* 300 bis 500 mg L⁻¹ beträgt; oder
(iv) der Wirkstoff gegen *Agrobacterium* Claforan ist; oder
(v) die Zeit, für die die embryogenen Kalli auf oder in dem somatische Embryonen induzierenden Medium kultiviert werden 10 bis 18 Tage, vorzugsweise 12 bis 16 Tage beträgt; oder
(vi) die Lichtbedingungen 20 bis26 µE m⁻² s⁻¹ sind; oder
(vii) die Photoperiode 14 bis 18 Stunden beträgt; oder
(viii) die Konzentration des Selektionswirkstoffs 10 bis 40 mg L⁻¹, eher vorzugsweise 20 bis 30 mg L⁻¹ beträgt.

18. Verfahren nach einem der vorstehenden Ansprüche, wobei in Schritt (f):
(i) die Konzentration des verwendeten Wirkstoffs gegen *Agrobacterium* 300 bis 700 mg L⁻¹, vorzugsweise 400 bis 600 mg L⁻¹ beträgt; oder
(ii) der Wirkstoff gegen *Agrobacterium* Claforan ist; oder
(iii) die somatischen Embryonen optional für weitere 2 bis 4 Stunden kultiviert werden.

## Revendications

1. Procédé pour produire un embryon somatique d'*Euphorbia pulcherrima* transgénique, comportant les étapes consistant à :
(a) mettre en culture un matériel végétal d'*Euphorbia pulcherrima* transformable, dans lequel le matériel végétal est une cellule de plante, un tissu de plante, un explant ou un protoplaste, sur ou dans un milieu d'induction de cal comportant CPA et BAP ;
(b) inoculer le matériel végétal d'*Euphorbia pulcherrima* transformable avec de l'*Agrobacterium* qui contient un gène hybride comportant un gène marqueur sélectionnable et un gène rapporteur et qui est capable de transférer le gène hybride au matériel végétal ;
(c) mettre en culture le matériel végétal d'*Euphorbia pulcherrima* transformable après inoculation avec *l'Agrobacterium* sur ou dans un milieu d'induction de cal comportant CPA et BAP en l'absence de l'agent de sélection et d'un agent anti-*Agrobacterium ;*
(d) mettre en culture le matériel végétal d'*Euphorbia pulcherrima* transformable sur ou dans un milieu d'induction de cal comportant CPA et BPA en présence d'un agent anti-*Agrobacterium* et de l'agent de sélection ;
(e) mettre en culture les cals embryogéniques ainsi produits sur ou dans un milieu d'induction d'embryons somatiques comportant NAA et 2iP en présence d'un agent anti-*Agrobacterium* et de l'agent de sélection ;
(f) mettre en culture les embryons somatiques ainsi produits sur ou dans un milieu d'induction d'embryons somatiques comportant NAA et 2iP en présence d'un agent anti-*Agrobacterium* et de l'agent de sélection, et mettre facultativement en sous-culture les embryons somatiques une ou plusieurs fois supplémentaires.

2. Procédé tel que revendiqué dans la revendication 1, lequel procédé comporte de plus l'étape consistant à :
(g) mettre en culture les embryons somatiques sur ou dans un milieu de maturation d'embryons somatiques comportant BAP.

3. Procédé tel que revendiqué dans la revendication 1 ou la revendication 2, lequel procédé comporte de plus l'étape consistant à :
(h) mettre en culture des plantules dérivées des embryons somatiques sur ou dans un milieu d'induction racinaire.

4. Procédé tel que revendiqué dans l'une quelconque des revendications précédentes, dans lequel l'*Euphorbia pulcherrima* est *Euphorbia pulcherrima* Willd. Ex Klotsch (poinsettia), de préférence dans lequel le cultivar d'*Euphorbia pulcherrima* est Millennium, Cristella, Angelika, Lilo ou Cortez.

5. Procédé tel que revendiqué dans l'une quelconque des revendications précédentes, dans lequel le matériel végétal est une cellule de plante, un cal, un embryon immature ou somatique, un méristème apical de pousse, un explant de feuille, un explant de tige ou un protoplaste, de préférence dans lequel le matériel végétal est un explant de tige.

6. Procédé tel que revendiqué dans l'une quelconque des revendications précédentes, dans lequel la plante à partir de laquelle le matériel végétal a été obtenu, est âgé de 6 à 8 semaines.

7. Procédé tel que revendiqué dans l'une quelconque des revendications précédentes, dans lequel à l'étape (a) :
(i) le milieu CI comporte de 0,1 à 0,35 mg 1⁻¹ de CPA ; ou
(ii) le milieu CI comporte de 0,1 à 0,35 mg 1⁻¹ de BAP ; ou
(iii) le matériel végétal est mis en culture sur ou dans un milieu d'induction de cal (CI) comportant CPA et BAP pendant 20 à 28 heures ; ou
(iv) le matériel végétal est mis en culture sur ou dans un milieu d'induction de cal (CI) comportant CPA et BAP dans l'obscurité ou sous des conditions de luminosité réduite.

8. Procédé tel que revendiqué dans l'une quelconque des revendications précédentes, dans lequel *l'Agrobacterium* est *Agrobacterium tumefaciens,* de préférence dans lequel *l'Agrobacterium* est *Agrobaeterium tumefaciens* souche LBA 4404.

9. Procédé tel que revendiqué dans l'une quelconque des revendications précédentes, dans lequel le gène hybride est un plasmide ou un vecteur d'ADN qui comporte un gène marqueur sélectionnable et un gène rapporteur flanqué d'au moins une bordure d'ADN-T.

10. Procédé tel que revendiqué dans l'une quelconque des revendications précédentes, dans lequel le gène hybride comporte :
(i) une séquence de bordure gauche d'ADN-T *d'Agrobacterium ;*
(ii) un gène marqueur sélectionnable comportant :
un promoteur qui fonctionne dans les cellules végétales d'*Euphorbia pulcherrima*
une séquence de nucléotides codant un polypeptide marqueur sélectionnable
une région 3' non traduite codant une séquence de terminaison
(iii) un gène rapporteur comportant :
un promoteur qui fonctionne dans des cellules végétales d'*Euphorbia pulcherrima*
une séquence de nucléotides présentant un intérêt une région 3' non traduite codant une séquence de terminaison
(iv) une séquence de bordure droite d'ADN-T *d'Agrobacterium.*

11. Procédé tel que revendiqué dans l'une quelconque des revendications précédentes, dans lequel le gène marqueur sélectionnable code un polypeptide qui confère une résistance à l'agent de sélection.

12. Procédé tel que revendiqué dans l'une quelconque des revendications précédentes, dans lequel l'agent de sélection est un antibiotique ou un herbicide, de préférence dans lequel le gène marqueur sélectionnable est la néomycine phosphotransférase II (nptII) et l'agent de sélection est la kanamycine.

13. Procédé tel que revendiqué dans l'une quelconque des revendications précédentes, dans lequel le gène rapporteur comporte :
- un gène de résistante à la maladie ou un gène codeur de protéines (PR) relié à la pathogenèse ;
- un gène qui code un polypeptide qui modifie un trait phénotypique connu ou introduit un nouveau trait phénotypique dans la plante ;
- un gène qui code un polypeptide qui modifie la couleur de la totalité ou d'une partie de la plante ou affecte la valeur ornementale de la plante ; ou
- une construction de silençage génique,
de préférence dans lequel le gène rapporteur comporte une molécule d'acide nucléique qui code un fragment de la protéine d'enrobage du virus de la mosaïque du poinsettia (PnMV) ou dans lequel le gène rapporteur comporte une molécule d'acide nucléique qui code un fragment d'une ARN polymérase ARN dépendante.

14. Procédé tel que revendiqué dans l'une quelconque des revendications précédentes, dans lequel :
(i) avant l'étape (b), les *Agrobacteria* se développent jusqu'à une valeur appropriée OD₆₀₀ = 0,5 à 1,0 ; ou
(ii) à l'étape (b), les *Agrobacteria* sont mis en co-culture avec le matériel végétal pendant 2 à 20 minutes, de manière plus préférée de 5 à 15 minutes et de manière la plus préférée environ 5 minutes.

15. Procédé tel que revendiqué dans l'une quelconque des revendications précédentes, dans lequel à l'étape (c) :
(i) les *Agrobacteria* sont mis en co-culture avec le matériel végétal à une température de 20 à 28 °C, de manière plus préférée de 22 à 26 °C ; ou
(ii) les *Agrobacteria* sont mis en co-culture avec le matériel végétal pendant 24 à 120 heures, de manière plus préférée pendant 48 à 96 heures ; ou
(iii) les *Agrobacteria* sont mis en co-culture avec le matériel végétal dans l'obscurité ou sous des conditions de luminosité réduite.

16. Procédé tel que revendiqué dans l'une quelconque des revendications précédentes, dans lequel à l'étape (d) :
(i) la concentration de l'agent *anti-Agrobacterium* est de 300 à 500 mg 1⁻¹ ; ou
(ii) l'agent *anti-Agrobacterium* est le claforan ; ou
(iii) la durée pendant laquelle le matériel végétal est mis en culture sur ou dans le milieu d'induction de cal (CI) est de 2 à 15 jours, de préférence de 5 à 12 heures, et de manière la plus préférée de 7 à 10 jours ; ou
(iv) les conditions de luminosité sont de 20 à 26 µE m⁻² s⁻¹ ; ou
(v) la photopériode est de 14 à 18 heures.

17. Procédé tel que revendiqué dans l'une quelconque des revendications précédentes, dans lequel à l'étape (e) :
(i) le milieu d'induction d'embryons somatiques comporte 0,1 à 0,4 mg 1⁻¹ de NAA, de préférence de 0,25 à 0,35 mg 1⁻¹ de NAA ; ou
(ii) le milieu d'induction d'embryons somatiques comporte de 0,10 à 0,30 mg 1⁻¹ de 2iP, de préférence de 0,12 à 0,20 mg 1⁻¹ de 2iP ; ou
(iii) la concentration de l'agent *anti-Agrobacterium* est de 300 à 500 mg 1⁻¹ ; ou
(iv) l'agent *anti-Agrobacterium* est le claforan ; ou
(v) la durée pendant laquelle les cals embryonnaires sont mis en culture sur ou dans le milieu d'induction d'embryons est de 10 à 18 jours, de préférence de 12 à 16 jours ; ou
(vi) les conditions de luminosité sont de 20 à 26 µE m⁻² s⁻¹ ; ou
(vii) la photopériode est de 14 à 18 heures ; ou
(viii) la concentration de l'agent de sélection est de 10 à 40 mg 1⁻¹, de manière plus préférée de 20 à 30 mg 1⁻¹.

18. Procédé tel que revendiqué dans l'une quelconque des revendications précédentes, dans lequel à l'étape (f) :
(i) la concentration de l'agent *anti-Agrobacterium* utilisé est de 300 à 700 mg 1⁻¹, de manière plus préférée de 400 à 600 mg 1⁻¹ ; ou
(ii) l'agent *anti-Agrobacterium* est le claforan ; ou
(iii) les embryons somatiques sont facultativement mis en sous-culture 2 à 4 fois supplémentaires.


### SEQUENCE LISTING

<110> Norwegian Institute for Agricultural and Environmental Research
<120> Transformation of plants
<130> 489.95248/01
<160> 6
<170> PatentIn version 3.3
<210> 1
   <211> 31
   <212> DNA
   <213> Unknown
<220>
   <223> Forward PCR primer used to amplify CP fragment
<400> 1
   acgtctagaa accacgtcga ctccactcca t 31
<210> 2
   <211> 29
   <212> DNA
   <213> Unknown
<220>
   <223> Reverse PCR primer used to amplify CP fragment
<400> 2
   agcatcgata gcttgccgct caccagcac 29
<210> 3
   <211> 34
   <212> DNA
   <213> Unknown
<220>
   <223> Forward PCR primer used to amplify R2 fragment
<400> 3
   acgtctagat ttagcaaaac gcagcacaaa atca 34
<210> 4
   <211> 32
   <212> DNA
   <213> Unknown
<220>
   <223> Reverse PCR primer used to amplify R2 fragment
<400> 4 32
   agcatcgatt ctccagacac catgattggg tg 32
<210> 5
   <211> 33
   <212> DNA
   <213> Unknown
<220>
   <223> Forward PCR primer used to amplify R3 fragment
<400> 5
   acgtctagat tcgctttaaa acagaaagca cca 33
<210> 6
   <211> 30
   <212> DNA
   <213> Unknown
<220>
   <223> Reverse PCR primer used to amplify R3 fragment
<400> 6
   agcatcgatg cctcgtagct tggttgggtt 30